# NEW EUROPEAN PATENT SPECIFICATION

(11) **EP 0 862 445 B2**
(45) Date of publication and mention of the opposition decision: **22.03.2017**
(45) Mention of the grant of the patent: 08.05.2013
(21) Application number: 96934086.8
(22) Date of filing: 07.10.1996
(51) Int. Cl.: A61K 35/76, A61K 45/06, A61P 35/00, A61K 48/00

(54) **Combination of herpes simplex virus and chemotherapy for treating cancer**
Kombination von Herpes simplex Virus und Chemotherapie für Krebstherapie
Combinaison de virus herpes simplex avec la chimithérapie pour le traitement du cancer

(30) Priority: 06.10.1995 US 540343
(43) Date of publication of application: 09.09.1998
(73) Proprietor: Arch Development Corporation, Chicago, IL 60637 (US); DANA-FARBER CANCER INSTITUTE, Boston, MA 02115 (US)
(72) Inventor: HALLAHAN, Dennis, E., Park Ridge, IL 60068 (US); WEICHSELBAUM, Ralph, R., Chicago, IL 60614 (US); KUFE, Donald, Wellesley, MA 02181 (US); SIBLEY, Gregory, S., Chapel Hill, NC (US); ROIZMAN, Bernard, Chicago, IL 60637 (US)
(74) Representative: Bösl, Raphael Konrad
(86) International application number: PCT/US1996/016047
(87) International publication number: WO 1997/012623

(56) References cited:
- EP-A1- 0 514 603
- EP-A1- 0 514 603
- WO-A1-96/00007
- WO-A2-95/20960
- WO-A2-97/03635
- CANCER RESEARCH, vol. 54, no. 9, 1 May 1994, BALTIMORE, MD, US, pages 2287-2291, XP002025050 T. FUJIWARA ET AL.: "INDUCTION OF CHEMOSENSITIVITY IN HUMAN LUNG CANCER CELLS IN VIVO BY ADENOVIRUS-MEDIATED TRANSFER OF THE WILD-TYPE p53 GENE."
- NATURE MEDICINE, vol. 1, no. 8, August 1995, NEW YORK, N.Y., US, pages 786-791, XP002024926 D.E. HALLAHAN ET AL.: "SPATIAL AND TEMPORAL CONTROL OF GENE THERAPY USING IONIZING RADIATION." cited in the application
- JOURNAL OF VIROLOGY, vol. 68, no. 12, December 1994, WASHINGTON, DC, US, pages 8304-8311, XP002024927 J. CHOU ET AL.: "DIFFERENTIAL RESPONSE OF HUMAN CELLS TO DELETIONS AND STOP CODONS IN THE GAMMA 1 34.5 GENE OF HERPES SIMPLEX VIRUS." cited in the application
- INTERNATIONAL JOURNAL OF CANCER, vol. 67, no. 3, 29 July 1996, GENEVA, CH, pages 386-392, XP000644437 M.V. BLAGOSKLONNY ET AL.: "IN VITRO EVALUATION OF A p53-EXPRESSING ADENOVIRUS AS AN ANTI-CANCER DRUG."
- FUJIWARA ET AL: 'Induction of Chemosensitivity in Human Lung Cancer cells in Vivo by Adenovirus-mediated Transfer of the Wild-Type p53 Gene' CANCER RESEARCH vol. 54, 01 May 1994, pages 2287 - 2291
- SIBLEY ET AL: 'Evaluation of a Gene Therapy System Using a Replication Competent, Non-Neurovirulent HSV-1 Viral Vector Used in Combination with Radiotherapy' INTERNATIONAL JOURNAL OF RADIATION ONCOLOGY BIOLOGY PHYSICS vol. 1, no. 9, September 1995, page 173
- KULU ET AL.: 'Concurrent chemotherapy inhibits Herpes simplex virus 1 replication and oncolysis' CANCER GENE THER. 20 February 2013,

## Description

### BACKGROUND OF THE INVENTION

### 1. Field of the Invention

The present invention relates generally to the fields of cell and tumor killing utilizing DNA damaging agents. More particularly, it concerns the use of selected viruses to enhance the effects of DNA damaging agents to kill cells and potentiate the therapeutic effect of these modalities.

### 2. Description of the Related Art

Recently, there has been a renewed interest in the potential use of cytolytic viruses in the treatment of cancer (Lorence *et al.*, 1994; Mineta *et al.*, 1994; Kenney *et al.*, 1994). The rationale for such an approach stems from case reports in the clinical literature describing tumor regression in human cancer patients during virus infection (Cassel *et al*., 1992). In one clinical trial, regression of tumors occurred in cancer patients treated with a wild-type mumps virus (Cassel *et al.*, 1992). In another report, complete remission occurred in a chicken farmer with widely metastatic gastric cancer during a severe outbreak of Newcastle disease (NDV) within the chicken population (Csatary, 1992).

Biomedical investigation has focused on the utilization of viruses as either direct therapeutics or for gene therapy, including the experimental therapy of brain tumors. For the experimental treatment of malignant gliomas, two approaches have predominated (Daumas-Duport *et al.*, 1988; Kim *et al.*, 1991; Culver *et al.*, 1992; Ram *et al.*, 1993(a); Ram *et al.*, 1993(b); Ram *et al.*, 1994) (Takamiya *et al.*, 1992; Martuza *et al.*, 1991; Maztuza *et al.*, 1991). The first involves deliberate *in situ* inoculation of cells infected with a retrovirus (producer cells) expressing the herpes simplex virus 1 (HSV-1) thymidine kinase gene into the tumor mass followed by treatment with ganciclovir (GCV), an antiviral drug (Culver *et al.*, 1992). The retrovirus is secreted from the producer cells and infects the tumor cells. GCV is selectively phosphorylated by the HSV-1 thymidine kinase to its mono-phosphate derivative and by cellular enzymes to a triphosphate derivative, which kills the tumor cells. Limitations of this approach include the quantity of nondividing cells that can be inoculated directly into the brain tumor, the relatively low yield of retroviruses, and the requirement for administration of GCV, a drug that has significant hematopoietic toxicity and does not penetrate the central nervous system to a great extent.

An alternative approach utilizes genetically engineered HSV. Among the mutants tested for this purpose were viruses lacking the thymidine kinase or ribonucleotide reductase gene or a genetically engineered virus lacking the γ34.5 gene (Markert *et al.*, 1993). Although some of the viruses tested to date prolonged the survival of tumor-bearing animals, none totally destroyed the tumor mass. Some of the deletion mutants tested, notably those that are thymidine kinase-negative, are potentially hazardous, since such viruses can cause encephalitis in animal models and are not treatable by drugs that depend on the viral thymidine kinase for their activity (Erlich *et al.*, 1989). The interest in testing of y34.5- viruses stems from studies on the function of the γ34.5 gene and the phenotype of these viruses carrying deletions and substitutions in that gene. The γ34.5 gene maps in the sequences flanking the long unique sequence and is present in two copies in the viral genome (Chou *et al.*, 1990; Ackermann *et al.*, 1986; Chou *et al.*, 1986). Mutants lacking both γ34.5 genes (*e.g.*, recombinant R3616) are apathogenic and fail to replicate in the central nervous system of mice (30). In cell culture, particularly in human fibroblasts and in the SK-N-SH human neuroblastoma cells, R3616 fails to prevent a stress response induced by the onset of viral DNA synthesis (Chou *et al.*, 1992). In consequence, protein synthesis is totally and prematurely shut off, resulting in cell death and significantly reduced viral yields. Although R3616 possesses many of the properties desired for cancer therapy, its effectiveness may be limited because its host range is very restricted.

While treatment with viruses alone or with DNA damaging agents alone provide some relief measure of cell killing, the overall cell death rate is generally below that obtained utilizing other treatment modalities. One type of cancer that would benefit from an increased therapeutic potential is malignant glioma. These cancers are the most common primary intracranial malignant tumor, accounting for 30% of primary brain tumors (Levin *et al.*, 1989). The estimated tumor incidence in the United States is 14.7 per 100 thousand, resulting in 5000 new cases annually (Mahaley *et al.*, 1989). In spite of aggressive surgical therapy, radiotherapy, and chemotherapy of patients with malignant gliomas, the overall 5-year survival is <5.5%, and the median survival is 52 weeks. This poor survival has remained virtually unchanged over the past 20 years (Levin *et al.*, 1989; Mahaley *et al.*, 1989; Salazar *et al.*, 1979; Walker *et al.*, 1980; Daumas-Duport *et al.*, 1988; Kim *et al.*, 1991). These abysmal survival rates have reinforced the need for new modalities of therapy. In view of such statistics, it would therefore be of great importance to develop methods of improving the therapeutic ability of current techniques of treating neoplastic disease.

### SUMMARY OF THE INVENTION

The present invention, in a general and overall sense, concerns the use of viruses in combination with chemotherapy to potentiate the therapeutic effect. In particular, the inventors have discovered that certain viruses, for example adenovirus and herpes simplex virus, act in an additive manner *in vitro* or, surprisingly and unexpectedly, in a synergistic manner *in vivo* to enhance cell killing following exposure to ionizing radiation. In particular, tumor cell growth is controlled using the methods and compositions of the invention. As used herein, tumor cell formation and growth describes the formation and proliferation of cells that have lost the ability to control cellular division, thus forming cancerous cells. Using the methods of the invention, a number of different types of transformed cells are potential targets for control, such as carcinomas, sarcomas, melanomas, gliomas, lymphomas, and a wide variety of solid tumors. While any tissue having malignant cell growth may be a target, brain, lung and breast tissue are preferred targets.

In one aspect, the invention concerns a combination of a herpes simplex virus (HSV) and an effective amount of a chemotherapeutic agent which induces DNA damage for simultaneous, separate or sequential use in a method of treatment of the human or animal body."

In another aspect, the invention concerns a use of a herpes simplex virus (HSV) for the manufacture of a medicament for the therapy of tumor, wherein the virus is administered simultaneously, separately or sequentially in combination with an effective amount of a chemotherapeutic agent which induces DNA damage."

In certain embodiments, the invention is a method of potentiating the response of a cell to chemotherapeutic agents that comprises first administering at least one virus to the cell, followed by exposing the cell to a chemotherapeutic agent. The viruses that are contemplated to be within the scope of the invention are Herpes Simplex Viruses. In exemplary embodiments, the virus is HSV-1. As used herein, "potentiate" means to increase the level of cell killing above that seen for a treatment modality alone. The potentiation may be additive, or it may be synergistic. The chemotherapeutic agent in a chemotherapeutic agent which induces DNA damage.

The use of ionizing radiation is also described. The radiation may be delivered by external sources, such as from gamma or beta sources, or it may be supplied from linear accelerators and the like. In other embodiments, the ionizing radiation may be delivered to a cell by radioisotopes or by providing a radiolabeled antibody that immunoreacts with an antigen of the tumor, followed by delivering an effective amount of the radiolabeled antibody to the tumor.

In addition to ionizing radiation, other DNA damaging agents are described. DNA damaging agents or factors are defined herein as any chemical compound or treatment method that induces DNA damage when applied to a cell. Such agents and factors include ionizing radiation and waves that induce DNA damage, such as, γ-irradiation, X-rays, UV-irradiation, microwaves, electronic emissions, and the like. The invention claims the use of a variety of chemical compounds, also described as "chemotherapeutic agents", which function to induce DNA damage, all of which are intended to be of use in the combined treatment methods disclosed herein. Chemotherapeutic agents contemplated to be of use, include, e.g., alkylating agents such as mitomycin C, adozelesin, *cis*-platinum, and nitrogen mustard. The invention also encompasses the use of a combination of one or more chemotherapeutic agents, which induce DNA damage, with one or more viruses.

The invention also contemplates methods of controlling cell growth by to a cell a virus that contains foreign DNA. The DNA may be in the form of a heterologous promoter sequence or it may be a heterologous gene encoding a structural protein. Also contemplated is a heterologous promoter sequence that is operatively linked to a structural gene coding for a tumoricidal gene, such as TNF-α. In certain methods, the tumor is first treated with a therapeutically effective amount of a virus that contains a DNA molecule comprising a radiation responsive enhancer-promoter operatively linked to an encoding region that encodes a polypeptide having the ability to inhibit growth of a tumor cell. Following uptake by the tumor cells, the tumor area is exposed to an effective expression-inducing dose of ionizing radiation that results in production of the protein.

To kill a cell in accordance with the present invention, one would generally contact the cell with a chemotherapeutic agent which induces DNA damage and a virus, such as HSV-1 in a combined amount effective to kill the cell. The term "in a combined amount effective to kill the cell" means that the amount of the DNA damaging agent and virus that are sufficient so that, when combined within the cell, cell death is induced. Although not required in all embodiments, the combined effective amount of the two agents will preferably be an amount that induces more cell death than the use of either element alone, and even one that induces synergistic cell death in comparison to the effects observed using either agent alone. A number of *in vitro* parameters may be used to determine the effect produced by the compositions and methods of the present invention. These parameters include, for example, the observation of net cell numbers before and after exposure to the compositions described herein.

Similarly, a "therapeutically effective amount" is an amount of a DNA damaging agent and a virus that, whm administered to an animal in combination, is effective to kill cells within the animal. This is particularly evidenced by the killing of cancer cells within an animal or human subject that has a tumor. The methods of the instant invention are thus applicable to treating a wide variety of animals, including mice and humans. "Therapeutically effective combinations" are thus generally combined amounts of DNA damaging agents and viruses or viral agents that function to kill more cells than either element alone and that reduce the tumor burden.

In certain embodiments, a process of enhancing cell death is provided, which comprising the steps of first treating cells or tumor tissue with a chemotherapeutic agent which induces DNA damage such as an alkylating agent, followed by contacting the cells or tumors with a herpes virus.

DNA damaging agents or factors are defined herein as any chemical compound or treatment method that induces DNA damage when applied to a cell. Such agents and factors include radiation and waves that induce DNA damage, such as, γ-irradiation, X-rays, UV-irradiation, microwaves, electronic emissions, and the like. A variety of chemical compounds, which may be described as "chemotherapeutic agents", also function to induce DNA damage, all of which are intended to be of use in the combined treatment methods disclosed herein. Chemotherapeutic agents contemplated to be of use, include, *e.g.*, mitomycin C (MMC), adozelesin, *cis*-platinum, nitrogen mustard, 5-fluarouracil (5FU), etoposide (VP-16), camptothecin, actinomycin-D, cisplatin (CDDP).

The invention provides, in certain embodiments, methods and compositions for killing a cell or cells, such as a malignant cell or cells, by contacting or exposing a cell or population of cells to one or more chemotherapeutic agents which induce DNA damage and one or more viruses inhibitors in a combined amount effective to kill the cell(s). Cells that may be killed using the invention include, *e.g.*, undesirable but benign cells, such as benign prostate hyperplasia cells or over-active thyroid cells; cells relating to autoimmune diseases, such as B cells that produce antibodies involved in arthritis, lupus, myasthenia gravis, squamous metaplasia, dysphasia and the like. Although generally applicable to killing all undesirable cells, the invention has a particular utility in killing malignant cells. "Malignant cells" are defined as cells that have lost the ability to control the cell division cycle, and exhibit uncontrolled growth and a "transformed" or "cancerous" phenotype.

It is envisioned that the cell that one desires to kill may be first exposed to a virus, and then contacted with the chemotherapeutic agent(s) which induce(s) DNA damage or *vice versa.* In such embodiments, one would generally ensure that sufficient time elapses, so that the two agents would still be able to exert an advantageously combined effect on the cell. In such instances, it is contemplated that one would contact the cell with both agents within about 12 hours of each other, and more preferably within about 6 hours of each other, with a delay time of only about 4 hours being most preferred. These times are readily ascertained by the skilled artisan.

The terms "contacted" and "exposed", when applied to a cell, are used herein to describe the process by which a herpesvirus, and a DNA damaging agent or factor are delivered to a target cell or are placed in direct juxtaposition with the target cell. To achieve cell killing, both agents are delivered to a cell in a combined amount effective to kill the cell, *i.e*., to induce programmed cell death or apoptosis. The terms, "killing", "programmed cell death" and "apoptosis" are used interchangeably in the present text to describe a series of intracellular events that lead to target cell death.

The present invention also provides advantageous compositions for use for treating cancer that, generally, comprise administering to an animal or human patient with cancer a therapeutically effective combination of a chemotherapeutic agent witch induces DNA damage and a virus. Chemical DNA damaging agents and/or viruses may be administered to the animal, often in close contact to the tumor, in the form of a pharmaceutically acceptable composition. Direct intralesional injection is contemplated, as are other parenteral routes of administration, such as intravenous, percutaneous, endoscopic, or subcutaneous injection. In certain embodiments, the route of administration may be oral.

In terms of contact with a DNA damaging agent, it is described that this may be achieved by irradiating the localized tumor site with ionizing radiation such as X-rays, UV-light, γ-rays or even microwaves. As claimed, the tumor cells may be contacted with the DNA damaging agent or a virus by administering to the animal a therapeutically effective amount of a pharmaceutical composition comprising a DNA damaging compound, such as mitomycin C, adozelesin, cis-platinum, and nitrogen mustard and/or a virus. A chemical DNA damaging agent may be prepared and used as a combined therapeutic composition, or kit, by combining it with a virus, as described above.

The described methods of enhancing the effectiveness of radiotherapy in a mammals comprises administering to that mammal an effective amount of a pharmaceutical composition that contains a virus. As used herein, a "pharmaceutical composition" means compositions that may be formulated for *in vivo* administration by dispersion in a pharmacologically acceptable solution or buffer. Suitable pharmacologically acceptable solutions include neutral saline solutions buffered with phosphate, lactate, Tris, and the like.

In certain embodiments of the invention, the number of virus particles that are contacted to a host are about 10³ to about 10¹⁴ virus particles. In other embodiments, the number of virus particles is about 10⁵ to about 10¹² virus particles, and in exemplary embodiments, the number of virus particles is between about 10⁸ to about 10¹¹ virus particles.

The invention further describes methods of assessing the cellular response to the effect of viral therapy in conjunction with exposure of cells to ionizing radiation that comprises first, growing cells in culture, which is followed by exposing the cells with a selected virus and to an effective dose of ionizing radiation. The response of the cells to this treatment modality may be assessed by techniques known in the art, such as cell survival assays or enzymatic assays of selected biomarker proteins. Suitable viruses include, for example, adenovirus, HSV-1, retrovirus, or NDV. The specificity of viral vectors may be selected to be preferentially directed to a particular target cell, such as by using viruses that are able to infect particular cell types. Naturally, different viral host ranges will dictate the virus chosen for gene transfer, and, if applicable, the likely foreign DNA that may be incorporated into the viral genome and expressed to aid in killing a particular malignant cell type.

In using viruses within the scope of the present invention, one will desire to purify the virus sufficiently to render it essentially free of undesirable contaminants, such as defective interfering viral particles or endotoxins and other pyrogens, so that it will not cause any undesired reactions in the cell, animal, or individual receiving the virus. A preferred means of purifying the vector involves the use of buoyant density gradients, such as cesium chloride gradient centrifugation.

Preferred viruses will be replication defective viruses in which a viral gene essential for replication and/or packaging has been deleted from the virus. The use of an adenovirus is described, wherein any gene, whether essential (*e.g*. E1A, E1B, E2 and E4) or non-essential (*e.g*. E3) for replication, may be deleted and replaced with foreign DNA, or not replaced. Techniques for preparing replication defective adenoviruses are well known in the art, as exemplified by Ghosh-Choudhury, *et al.*, 1987. It is also well known that various cell lines may be used to propagate recombinant adenoviruses, so long as they complement any replication defect that may be present. A possible cell line is the human 293 cell line, but any other cell line that is permissive for replication, *e.g*. that expresses E1A and E1B, may be employed. Further, the cells may be propagated either on plastic dishes or in suspension culture in order to obtain virus stocks.

The patent does not only describe E1-lacking virus and E1 expressing cells. Other complementary combinations of viruses and host cells may be employed in connection with the present invention. Where a gene that is not essential for replication is deleted and replaced, such as, for example, the E3 gene, this defect will not need to be specifically complemented by the host cell. The adenovirus may be of any of the 42 different known serotypes or subgroups A-F. Adenovirus type 5 of subgroup C is the preferred starting material in order to obtain the conditional replication-defective adenovirus vector for use on the method of the present invention.

The uses and compositions of the present invention are suitable for killing a cell or cells both *in vitro* and *in vivo*. When the cells are to be killed are located within an animal, for example in an organ, the virus and the DNA damaging agent will be administered to the animal in a pharmacologically acceptable form. Direct intralesional injection of a therapeutically effective amount of a virus and/or a DNA damaging agent into a tumor site is one preferred method. Other parenteral routes of administration, such as intravenous, percutaneous, endoscopic, or subcutaneous injection are also contemplated.

As set forth above, any number of *in vitro* parameters may be used to determine the effect produced by the compositions and methods of the present invention. These parameters include, for example, the observation of net cell numbers before and after exposure to the disclosed treatment methods. Also, one may be able to assess the size of cells grown in culture, such as those colonies formed in tissue culture. Alternatively, one may measure parameters that are indicative of a cell that is undergoing programmed cell death such as, for example, the fragmentation of cellular genomic DNA into nucleoside size fragments, generally identified by separating the fragments by agarose gel electrophoresis, staining the DNA, and comparing the DNA to a DNA size ladder.

One may also use other means to assess cell killing. As set forth in the instant examples, one may measure the size of the tumor, either by the use of calipers, or by the use of radiologic imaging techniques, such as computerized axial tomography (CAT) or nuclear magnetic resonance (NMR) imaging.

In other embodiments of the invention, kits for use in killing cells, such as malignant cells, are contemplated. These kits will generally include, in a suitable container means, a pharmaceutical formulation of a virus for contacting the host cells. In certain kit embodiments, the chemotherapeutic agent which induces DNA damage, such as a DNA alkylating agent may be included in the kit. The kit components may be provided as a liquid solution, or a dried powder. A preferred approach is to provide a sterile liquid solution.

It is described that the combination of viral infection with radiation treatment produces tumor cures which are greater than those produced by treatment with radiation alone. Viral infection alone actually had no effect on cell killing, whether the virus contained an foreign gene insert or a either modality alone. Cells that contain genetic constructs constitutively producing toxins and are targeted with ionizing radiation provides a new conceptual basis for increasing the therapeutic ratio in cancer treatment.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.
FIG. 1 Shows U-87MG glioblastoma cell growth in hindlimbs of mice following exposure to HSV-1, radiation, and radiation plus HSV-1. Also shown is the effect ganciclovir on tumor volume, when given in combination with virus or virus plus radiation.
FIG. 2 Shows the regression rate of large tumors compared to small xenografts following treatment with radiation alone or adenovirus construct Ad.Egr-TNF plus radiation.
FIG. 3 Shows the regression rate of large tumors compared to small xenografts following treatment with radiation alone or adenovirus construct Ad.LacZ plus radiation.

### DETAILED DESCRIPTION OF THE PREFERRED EMBODIMENTS

The present invention presents methods that are a novel combination of viral infection and chemotherapy that act together to enhance cell killing *in vitro* and *in vivo.*

### VIRUSES

### Adenovirus (not claimed)

Adenoviruses have been widely studied and well-characterized as a model system for eukaryotic gene expression. Adenoviruses are easy to grow and manipulate, and they exhibit broad host range *in vitro* and *in vivo.* This group of viruses may be obtained in a highly infective state and at high titers, *e.g.*, 10⁹-10¹¹ plaque-forming unit (PFU)/ml. The Adenovirus life cycle does not require integration into the host cell genome, and foreign genes delivered by these vectors are expressed episomally, and therefore, generally have low genotoxicity to host cells. Adenoviruses appear to be linked only to relatively mild diseases, since there is no known association of human malignancies with Adenovirus infection. Moreover, no side effects have been reported in studies of vaccination with wild-type Adenovirus (Couch *et al.*, 1963; Top *et al.*, 1971), demonstrating their safety and therapeutic potential as *in vivo* gene transfer vectors.

Adenovirus vectors have been successfully used in eukaryotic gene expression (Levrero *et al.*, 1991; Gomez-Foix *et al.*, 1992) and vaccine development (Grunhaus and Horwitz, 1992; Graham and Prevec, 1992). Recently, animal studies demonstrated that recombinant Adenoviruses could be used for gene therapy (Stratford-Perricaudet and Perricaudet, 1991; Stratford-Perricaudet *et al.*, 1990; Rich *et al.*, 1993). Successful studies in administering recombinant Adenovirus to different tissues include trachea instillation (Rosenfeld *et al.*, 1991; Rosenfeld *et al.*, 1992), muscle injection (Ragot *et al.*, 1993), peripheral intravenous injection (Herz and Gerard, 1993), and stereotactic inoculation into the brain (Le Gal La Salle *et al.*, 1993).

Generation and propagation of the current Adenovirus vectors depend on a unique helper cell line, 293, which was transformed from human embryonic kidney cells by AD5 DNA fragments and constitutively expresses E1 proteins (Graham, *et al.*, 1977). Since the E3 region is dispensable from the Adenovirus genome (Jones and Shenk, 1978), the current Adenovirus vectors, with the help of 293 cells, carry foreign DNA in either the E1, the E3 or both regions (Graham and Prevec, 1991). In nature, Adenovirus can package approximately 105% of the wild-type genome (Ghosh-Choudhury, *et al.*, 1987), providing capacity for about 2 extra kb of DNA. Combined with the approximately 5.5 kb of DNA that is replaceable in the E1 and E3 regions, the maximum capacity of the current Adenovirus vector is under 7.5 kb, or about 15% of the total length of the vector. More than 80% of the Adenovirus viral genome remains in the vector backbone and is the source of vector-borne cytotoxicity.

As used herein, the term "recombinant" cell is intended to refer to a cell into which a recombinant gene, such as a gene from the adenoviral genome has been introduced. Therefore, recombinant cells are distinguishable from naturally occurring cells that do not contain a recombinantly introduced gene. Recombinant cells are thus cells having a gene or genes introduced through the hand of man. Within the present disclosure, the recombinantly introduced genes encode radiation sensitizing or radiation protecting factors and are inserted in the E1 or E3 region of the adenovirus genome. It is recognized that the present disclosure also encompasses genes that are inserted into other regions of the adenovirus genome, for example the E2 region.

It is understood that the adenovirus vector construct may therefore, comprise at least 10 kb or at least 20 kb or even about 30 kb of heterologous DNA and still replicate in a helper cell. By "replicate in a helper cell," it is meant that the vector encodes all the necessary *cis* elements for replication of the vector DNA, expression of the viral coat structural proteins, packaging of the replicated DNA into the viral capsid and cell lysis, and further that the *trans* elements are provided by the helper cell DNA. Replication is determined by contacting a layer of uninfected cells with virus particles and incubating said cells. The formation of viral plaques, or cell free areas in the cell layers is indicative of viral replication. These techniques are well known and routinely practiced in the art. It is understood that the adenoviral DNA that stably resides in the helper cell may comprise a viral vector such as an Herpes Simplex virus vector, or it may comprise a plasmid or any other form of episomal DNA that is stable, non-cytotoxic and replicates in the helper cell.

Heterologous DNA may be introduced into the viral genome. By heterologous DNA is meant DNA derived from a source other than the adenovirus genome, which provides the backbone for the vector. This heterologous DNA may be derived from a prokaryotic or a eukaryotic source such as a bacterium, a virus, a yeast, a plant or animal. The heterologous DNA may also be derived from more than one source. For instance, a promoter may be derived from a virus and may control the expression of a structural gene from a different source such as a mammal. Preferred promoters include viral promoters such as the SV40 late promoter from simian virus 40, the Baculovirus polyhedron enhancer/promoter element, RSV, Herpes Simplex Virus thymidine kinase (HSV tk), the immediate early promoter from cytomegalovirus (CMV) and various retroviral promoters including LTR elements.

The promoters and enhancers that may comprise the heterologous DNA will be those that control the transcription of protein encoding genes in mammalian cells may be composed of multiple genetic elements. The term promoter, as used herein refers to a group of transcriptional control modules that are clustered around the initiation site for RNA polymerase II. Promoters are believed to be composed of discrete functional modules, each comprising approximately 7-20 bp of DNA, and containing one or more recognition sites for transcriptional activator proteins. At least one module in each promoter functions to position the start site for RNA synthesis. The best known example of this is the TATA box, but in some promoters lacking a TATA box, such as the promoter for the mammalian terminal deoxynucleotidyl transferase gene and the promoter for the SV 40 late genes, a discrete element overlying the start site itself helps to fix the place of initiation.

Additional promoter elements regulate the frequency of transcriptional initiation. Typically, these are located in the region 30-110 bp upstream of the start site, although a number of promoters have recently been shown to contain functional elements downstream of the start site as well. The spacing between elements is flexible, so that promoter function is preserved when elements are inverted or moved relative to one another. Depending on the promoter, it appears that individual elements can function either cooperatively or independently to activate transcription.

The heterologous DNA may also comprise an enhancer. The basic distinction between enhancers and promoters is operational. An enhancer region as a whole must be able to stimulate transcription at a distance, which is not necessarily true of a promoter region or its component elements. On the other hand, a promoter must have one or more elements that direct initiation of RNA synthesis at a particular site and in a particular orientation, whereas enhancers lack these specificities. Aside from this operational distinction, enhancers and promoters are very similar entities. They have the same general function of activating transcription in the cell. They are often overlapping and contiguous, often seeming to have a very similar modular organization. Taken together, these considerations suggest that enhancers and promoters are homologous entities and that the transcriptional activator proteins bound to these sequences may interact with the cellular transcriptional machinery in fundamentally the same way. It is understood that any such promoter or promoter/enhancer combination may be included in the heterologous DNA of the adenoviral vector to control expression of the heterologous gene regions.

The heterologous DNA may include more than one structural gene under the control of the same or different promoters. The heterologous DNA may also include ribosome binding sites and polyadenylation sites or any necessary elements for the expression of the DNA in a eukaryotic or a mammalian cell. These vector constructs are created by methods well known and routinely practiced in the art such as restriction enzyme digestion followed by DNA ligase directed splicing of the various genetic elements. The heterologous DNA may further comprise a constitutive promoter. A constitutive promoter is a promoter that exhibits a basal level of activity that is not under environmental control. Some examples of constitutive promoters that may possibly be included as a part of the present invention include, but are not limited to, intermediate-early CMV enhancer/promoter, RSV enhancer-promoter, SV40 early and SV-40 late enhancer/promoter, MMSV LTR, SFFV enhancer/promoter, EBV origin of replication, or the Egr enhancer/promoter. However, it is understood that any constitutive promoter may be used in the practice of the invention and all such promoters/enhancers would fall within the spirit and scope of the claimed invention.

Another type of promoter that may comprise a portion of the heterologous DNA is a tissue specific promoter. A tissue specific promoter is a promoter that is active preferentially in a cell of a particular tissue type, such as in the liver, the muscle, endothelia and the like. Some examples of tissue specific promoters that may be used in the practice of the invention include the RSV promoter to be expressed in the liver or the surfactin promoter to be expressed in the lung, with the muscle creatine kinase enhancer combined with the human cytomegalovirus immediate early promoter being the most preferred for expression in muscle tissue, for example.

### Herpes Simplex Virus

Also described herein a method using HSV-1 for enhancing radiation control of tumors. While these viruses have been used in gene therapy, generally by inserting a therapeutic gene into the HSV-1 viral genome and transfecting into a cell, the present invention does not require the use of specific inserts for function. As used in the present invention, the virus, which has been rendered non-pathogenic, is combined with a pharmacologically acceptable carrier in order to form a pharmaceutical composition. This pharmaceutical composition is then administered in such a way that the mutated virus can be incorporated into cells at an appropriate area.

The use of the HSV-1 virus with a specific mutation in the γ₁34.5 gene provides a method of therapeutic treatment of tumorigenic diseases both in the CNS and in all other parts of the body (Chou 1992). The "γ₁34.5 minus" virus can induce apoptosis and thereby cause the death of the host cell, but this virus cannot replicate and spread (Chou 1992). Therefore, given the ability to target tumors within the CNS, the γ₁34.5 minus virus has proven a powerful therapeutic agent for hitherto virtually untreatable forms of CNS cancer. Furthermore, use of substances, other than a virus, which inhibit or block expression of genes with anti-apoptotic effects in target tumor cells can also serve as a significant development in tumor therapy and in the treatment of herpes virus infection, as well as treatment of infection by other viruses whose neurovirulence is dependent upon an interference with the host cells' programmed cell death mechanisms. The procedures to generate the above recombinant viruses are those published by Post and Roizman (1981), and U.S. Patent No. 4,769,331 incorporated herein by reference. Other viruses that are described herein include, but are not limited to, NDV, adeno-associated virus (AAV), and human papilloma virus (HPV). The currently preferred virus for use in the present invention is HSV-1.

For example, NDV injected into a primary cervical carcinoma produced tumor regression at the site of injection (Cassel *et al.*, 1965). NDV treatment caused partial tumor regression in 8 of 33 patients studied in a small clinical trial (Csatary *et al.*, 1993). NDV is directly cytotoxic to a wide variety of human cancer cells but not to normal fibroblasts *in vitro* (Lorence *et al.*, 1994; Reichard *et al.*, 1992). NDV is a potent inducer of tumor necrosis factor-α and NDV-infected cells are dramatically more sensitive to lysis by this cytokine than are uninfected cells (Chou *et al.*, 1992). A single local injection of NDV strain 73-T causes long-lasting complete regression of human neuroblastoma xenografts in athymic mice (Levin *et al.*, 1989). NDV is also cytotoxic to human tumors including HT1080 fibrosarcoma xenografts (Lorence *et al.*, 1994; Reichard *et al.*, 1993). Thus, it is described NDV and other cytotoxic viruses will be useful.

Genetically engineered HSV mutants can be used for the specific purpose of treatment of brain tumors without the requirement for alternative therapies (antiviral drugs) or the risk of progressive disease (Chambers, R, 1995). While the usefulness of the y34.5- virus has been demonstrated in another model (Takamiya *et al.*, 1992), the inventors show that the virus in which the γ34.5 gene is interrupted by a stop codon (R4009) rather than by deletion (R3616) appears to be more efficient in destroying tumor cells. The inventors attribute this greater survival benefit to enhanced replication competence of R4009 as compared to R3616. One explanation of this observation is that a low level of stop codon suppression takes place and that the low level of expression of γ34.5 enables the virus to effectively destroy tumor cells and yet not multiply to a level where it can cause encephalitis. The key to the development of effective oncolytic viruses may well depend on precise control of the expression of the γ34.5 gene, and this observation may be exploited to construct still more effective viruses. Recently, other laboratories have assessed the value of alterations at other sites within the HSV genome for the creation of viruses suitable for treatment of brain tumors (Mineta *et al.*, 1994).

In certain embodiments of the invention, foreign DNA is inserted into the viral genome. This foreign DNA may be a heterologous promoter region, a structural gene, or a promoter operatively linked to such a gene. Representative promoters include, but are not limited to, the CMV promoter, LacZ promoter, or Egr promoter.

### RETROVIRUSES (not claimed)

Alternatively, the vehicle may be a virus or an antibody that specifically infects or immunoreacts with an antigen of the tumor. Retroviruses used to deliver the constructs to the host target tissues generally are viruses in which the 3' LTR (linear transfer region) has been inactivated. These are enhancerless 3'LTR's, often referred to as SIN (self-inactivating viruses) because after productive infection into the host cell, the 3'LTR is transferred to the 5' end, and both viral LTR's are inactive with respect to transcriptional activity. Use of these viruses well known to those skilled in the art is to clone genes for which the regulatory elements of the cloned gene are inserted in the space between the two LTR's. An advantage of a viral infection system is that it allows for a very high level of infection into the appropriate recipient cell, *e.g.*, LAK cells.

For purposes of this invention, a radiation responsive enhancer-promoter that is 5' of the appropriate encoding region may be cloned into the virus using standard techniques well known in the art. Exemplary methods of cloning are set forth in Sambrook *et al.*,

### IONIZING RADIATION (not claimed)

Ionizing radiation causes DNA damage and cell killing, generally proportional to the dose rate. Ionizing radiation has been postulated to induce multiple biological effects by direct interaction with DNA or through the formation of free radical species leading to DNA damage (Hall. 1988). These effects include gene mutations, malignant transformation, and cell killing. Although ionizing radiation has been demonstrated to induce expression of certain DNA repair genes in some prokaryotic and lower eukaryotic cells, little is known about the effects of ionizing radiation on the regulation of mammalian gene expression (Borek, 1985). Several studies have described changes in the pattern of protein synthesis observed after irradiation of mammalian cells. For example, ionizing radiation treatment of human malignant melanoma cells is associated with induction of several unidentified proteins (Boothman, *et al.*, 1989). Synthesis of cyclin and co-regulated polypeptides is suppressed by ionizing radiation in rat REF52 cells but not in oncogene-transformed REF52 cell lines (Lambert and Borek, 1988). Other studies have demonstrated that certain growth factors or cytokines may be involved in x-ray-induced DNA damage. In this regard, platelet-derived growth factor is released from endothelial cells after irradiation (Witte, *et al.*, 1989).

In the present patent the term "ionizing radiation" means radiation comprising particles or photons that have sufficient energy or can produce sufficient energy via nuclear interactions to produce ionization (gain or loss of electrons). An exemplary and preferred ionizing radiation is an x-radiation. Means for delivering x-radiation to a target tissue or cell are well known in the art. Also, the phrase "effective expression-inducing dose of ionizing radiation" means that dose of ionizing radiation needed to stimulate or turn on a radiation responsive enhancer-promoter that is one embodiment of the present invention. The amount of ionizing radiation needed in a given cell generally depends upon the nature of that cell. Typically, an effective expression-inducing dose is less than a dose of ionizing radiation that causes cell damage or death directly. Means for determining an effective amount of radiation are well known in the art. The amount of ionizing radiation needed in a given cell naturally depends upon the nature of that cell. As also used herein, the term "an effective dose" of ionizing radiation means a dose of ionizing radiation that produces an increase in cell damage or death when given in conjunction with a virus.

In a certain embodiments, an effective expression inducing amount is from about 2 to about 30 Gray (Gy) administered at a rate of from about 0.5 to about 2 Gy/minute. Even more preferably, an effective expression inducing amount of ionizing radiation is from about 5 to about 15 Gy. In other embodiments, doses of 2-9 Gy are used in single doses. An effective dose of ionizing radiation may be from 10 to 100 Gy, with 15 to 75 Gy being preferred, and 20 to 50 Gy being more preferred.

Any suitable means for delivering radiation to a tissue may be employed in the present invention in addition to external means. For example, radiation may be delivered by first providing a radiolabeled antibody that immunoreacts with an antigen of the tumor, followed by delivering an effective amount of the radiolabeled antibody to the tumor. In addition, radioisotopes may be used to deliver ionizing radiation to a tissue or cell.

### MATERIALS AND METHODS

**Cells and Viruses.** HSV-1(F) is the prototype wild-type HSV-1 strain used in the inventors' laboratories (Ejercito *et al.*, 1968). R3616 lacks 1000 bp from the coding domain of each copy of the γ34.5 gene. All viruses were grown and titered in Vero cells as described (Chou *et al.*, 1992; Chou *et al.*, 1990). The infected cells were disrupted by sonication, and the virus contained in the supernatant fluid after centrifugation at 1200 × g for 20 min was stored at -70°C.

The U87 glioma cell line was established from a human glioma tissue culture medium. Tumor cells were loaded into a 250µl Hamilton syringe fitted with a 30-gauge 0.5-inch needle, attached to a repeating dispenser, and mounted in a stereotaxic holder.

Animal studies were done in accordance with guidelines for care by The University of Chicago Committee on Animal Care. All animal studies were performed in accordance with acceptable federal standards.

**Statistical Analyses.** Kaplan-Meier survival data were analyzed with a computer software program. To estimate significance of differences in the median survivals by the log rank and Peto-Wilcoxon nonparametric hypothesis tests. The x2 distribution was used to compute the probability, p, as determined at a significance level of <0.01.

The following examples are included to demonstrate preferred embodiments of the invention. It should be appreciated by those of skill in the art that the techniques disclosed in the examples that follow represent techniques discovered by the inventor to function well in the practice of the invention, and thus can be considered to constitute preferred modes for its practice. However, those of skill in the art should, in light of the present disclosure, appreciate that many changes can be made in the specific embodiments that are disclosed and still obtain a like or similar result without departing from the spirit and scope of the invention.

### Reference EXAMPLE I

### Viral Enhancement of Tumor Control With Radiation Therapy

### Background

The present inventors have utilized viral vectors to deliver gene therapy to human tumor xenografts (Hallahan *et al.*, 1995; Sibley *et al.*, 1995). Vector without the TNF gene was used as a control in the treatment of these tumors. These studies indicated that the viral vectors enhanced tumor control by x-irradiation.

### HERPES SIMPLEX VIRUS (HSV)

The objective of the studies described here was to establish a model of malignant glioma in mice and to compare the effectiveness of R3616 and R4009 in treatment of this tumor in the murine model.

### METHODS:

### Viruses

R3616 was created by a deletion of the gene conferring neurovirulence, γ34.5. An Egr-TNF-α construct was also created by ligating the Egr-1 enhancer/promoter region upstream to the human TNF-α gene, which construct was then inserted into the γ34.5 locus by recombination. This modified virus was designated R899-6.

### Growth of human xenografts in vivo:

U-87MG glioblastoma cells were obtained from the American Type Culture Collection (ATCC) and cultured *in vitro* using standard procedures. An injection of 5 × 106 cells in 10 µl was given subcutaneously in the hind limb of a nude mouse. When the tumor volume was greater than 100mm³, and preferably reached approximately 300mm³, the tumor was removed and minced into pieces measuring 2 mm in greatest dimension. These pieces were implanted subcutaneously right into the hind limb of anesthetized nude mice through a small incision. Tumors were randomized to a treatment group when the tumors reached 100 mm³ (but not greater than 300 mm³). Tumor volumes were calculated by the formula "a × b × c/2" that is an approximation of the formula for an ellipsoid (πd³/6). Tumors were measured twice weekly using vernier calipers.

### Viral Injection:

Viral stock solutions with a titer of 2 × 109 PFU/ml were used. The virus was tittered *in vitro* using Vero cells as previously described. Mice assigned to the virus alone or virus plus RT groups received an inoculation of 10 ml, or 2 × 107 PFU, on the first day of treatment (Group 1) or the first three days of treatment (Group 2) using a Hamilton syringe. Each inoculation was given *via* three injections to improve viral distribution in the tumor.

### Radiotherapy:

Mice assigned to the radiotherapy alone or virus plus RT group were irradiated the first two days of treatment. A dose of 20 Gy was delivered on day 1 and 25 Gy on day 2. Mice were immobilized in lucite chambers and the right hind limb was extended and taped. A lead shield was then placed over the body with a cutout portion to provide a margin around the tumor. Irradiation was given with 250 KV photons with a 0.5 mm Cu filter at a dose rate of 1.88 Gy/min using a Maxitron generator.

### Quantitation of TNF-α

TNF-α levels were quantitated and localized by ELISA, using the method described by Weichselbaum *et al.* (1994).

### RESULTS:

### Study 1

*In vitro* infection with 1 × 10⁶ PFU of R899-6 resulted in significantly greater TNF-α production than in uninfected control U-87 cells, seen at 4-6 hours with a peak at 12 hours following viral infection. TNF-α production was also demonstrated *in vivo* in the hindlimbs of mice. The *in vitro* survival studies showed R899-6 and R3616 to be equally cytotoxic. In addition, the addition of single radiation doses of 2-9 Gy was additive, or in some cases, synergistic for these viruses. The mean tumor volumes for 6 groups, 8 mice each, is shown in Table 1, 21 days post treatment.

**TABLE 1**

| Treatment Group | Mean Tumor Vol. (mm³) | TNF-α (pg/plate) |
|---|---|---|
| Control | 2772±585 | 65 |
| R899-6 | 376±192 | 3164 |
| R3616 | 1264±449 | 70 |
| RT Alone | 262±49 | -- |
| R3616 + RT | 111±31 | -- |
| R899-6 + RT | 74±22 | -- |

### Study 2

A total of 98 nude mice were treated on the 6 treatment arms, of which 8 died of treatment-unrelated causes and were censored from analysis (1 R899-6, 2 R3616, 4 RT alone, 1 R3616 + RT). The results are presented in Table 2. All of the control mice were sacrificed at a median time of 21 days (range 10-35 days) due to excessive tumor growth (> 2000 mm³). In the virus alone groups, 25%, and 20% of the tumors were reduced or controlled in the R899-6 and R3616 groups, respectively, while the remainder grew to > 2000 mm³. In the RT alone group a minority (12.5%) were reduced or controlled, whereas the remainder had persistent tumor out to 90 days. Conversely, the majority of tumors in the combined treatment arms were reduced or controlled. Specifically, 62.5% of the R3616 + RT and 64.7% of the R899-6 + RT were killed (Tables 2 and 3).

**TABLE 2**

| **Treatment** | **Results** | | | |
|---|---|---|---|---|
| | Sacrificed¹ | Controlled² | Neither | Total |
| Control | 10 | 0 | 0 | 10 |
| R899-6 | 12 | 4 | 0 | 16 |
| R3616 | 12 | 3 | 0 | 15 |
| RT Alone | 0 | 2 | 14 | 16 |
| R3616 + RT | 0 | 10 | 6 | 16 |
| R899-6 + RT | 2 | 11 | 4 | 17 |
| Total | 36 | 30 | 24 | 90 |
| ¹ Mice sacrificed when tumors exceeded 2000 mm³. | | | | |
| ²"Controlled" is defined as × 10% of day 0 tumor volume. | | | | |

**TABLE 3**

| **Study #1 (Single Injection)** | | | |
|---|---|---|---|
| | n | Controlled (%) | Uncontrolled (%) |
| Control | 10 | 0 | 100 |
| R899-6 | 17 | 23.5 | 76.5 |
| R3616 | 15 | 20 | 80 |
| RT Alone | 16 | 25 | 12.5 |
| R3616 + RT | 16 | 62.5 | 0 |
| R899-6 + RT | 16 | 68.8 | 12.5 |

**TABLE4**

| **Group 3** | | | |
|---|---|---|---|
| **Three injections of virus plus radiation** | | | |
| | n | Controlled (%) | Uncontrolled (%) |
| Control | 12 | 0 | 100 |
| R899-6 | 12 | 0 | 38 |
| R3616 | 12 | 0 | 33 |
| RT Alone | 12 | 0 | 0 |
| R3616 + RT | 12 | 33 | 0 |
| R899-6 + RT | 12 | 0 | 0 |

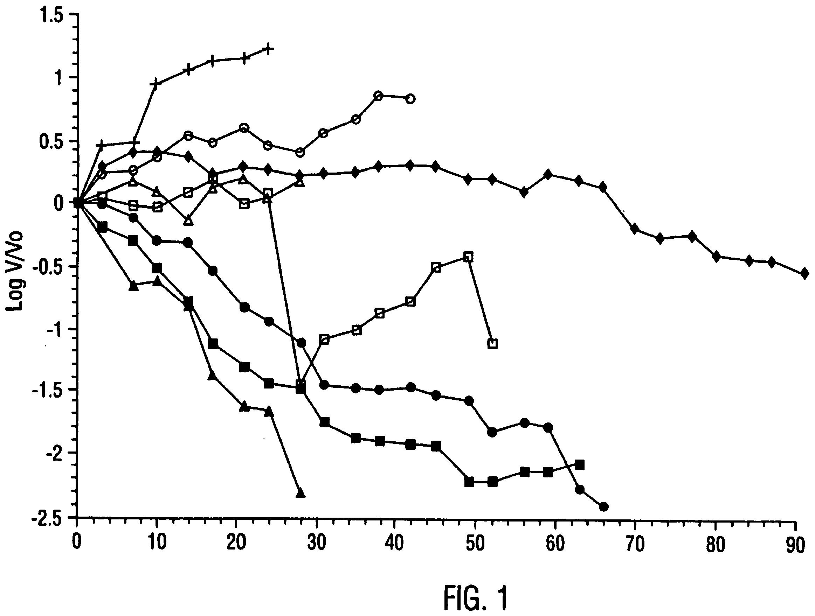

FIG. 1 depicts U-87MG glioblastoma cell growth in hindlimbs of mice following exposure to HSV-1 (3X), radiation (RT), and radiation plus HSV-1. Control animals (vertical tick) were sacrificed after 24 days when tumor volume reached >2000 mm³. Radiotherapy alone reduced or controlled only a small number of tumors out to 90 days; the volumes remaining were relatively unchanged from day 1 post treatment. The majority of tumors in the study arms that combined treatment of radiotherapy with virus (899.6 + RT (closed circles) and 3616 + RT (closed squares) were reduced or controlled.

When virus was given in combination with ganciclovir (3616 + GCV (open triangles)), the results were similar as those for radiation alone. However, 3636 + GCV and radiation showed even greater effectiveness (closed triangles). he first involves deliberate *in situ* inoculation of cells infected with a retrovirus (producer cells) expressing the herpes simplex virus 1 (HSV-1) thymidine kinase gene into the tumor mass followed by treatment with ganciclovir (GCV), an antiviral drug

### Reference EXAMPLE II

### Adenovirus Type 5 Enhances Tumor Control By X-Irradiation

The replication deficient adenovirus type 5 (Ad5) genome (McGrory *et al.*, 1988; Jones *et al.*, 1979) has been shown to infect human epithelial carcinoma cells (Hallahan, 1995; O'Malley, 1995).

In the present study, human colon carcinoma cells (10⁶ WiDr cells) were injected into the hindlimbs of nude mice and tumors were grown to a mean tumor volume of 260 mm³. Xenografts were injected with 2×10⁸ PFU of Ad5.null, two injections per week for a total of four weeks. As used herein, AD5.null is the replication deficient adenovirus type 5 that does not contain foreign genes to be expressed, such as therapeutic genes. Control tumors were either not injected or injected with an equivalent volume of buffered saline. An Ela, partial Elb, partial E3"Ad5 based adenovirus vector was modified to contain an expression cassette that replaced the E₁ region with the Egr enhancer/promoter coupled to the gene encoding TNF-α.

Irradiated mice were immobilized in lucite chambers and the entire body was shielded with lead except for the tumor bearing hindlimb. Tumors were irradiated with 5 Gy per day, 4 days per week, to a total dose of 50 Gy using a Maxitron generator (1.88 Gy/min). Tumor volumes were calculated by the formula (a × b × c/2) that was derived from the formula for an ellipsoid (πd³/6). Data were calculated as the percent of original (day 0) tumor volume and are presented as the fractional tumor volume ±SEM versus days post treatment.

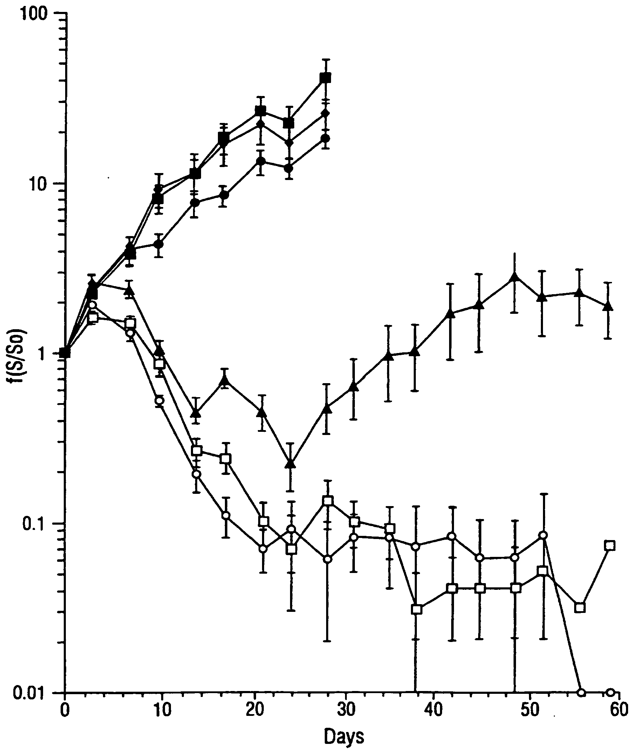

The regression rate of large (>260 mm³) original tumor volume was compared to small (<260 mm³) xenografts following treatment with radiation alone or Ad.Egr-TNF plus radiation. This tumor volume was selected because it is the median in this study. Control tumors were either not injected or injected with an equivalent volume of buffered saline. As seen in FIG. 2, control tumors (closed squares) grew until the tumor volume reached > 2000 mm³, (at approximately 28 days) at which point the mice were sacrificed due to excessive tumor growth. The same was true for mice receiving the Ad.Null (closed diamonds) or Ad.Egr-TNF (closed circles). Treatment with 45 Gy ionizing radiation caused an initial drop in the fractional tumor volume, which gradually increased to remain relatively constant at the pretreatment volume (closed triangles). Radiation plus Ad.Null (open circles) or Ad.Egr-TNF (open squares), however, enhanced cell killing synergistically over that seen for radiation alone.

**Ad LacZ Null:** In studies similar to those set forth above, the LacZ reporter gene (AD5-LacZ) was integrated in the by substituting the E₁ region with the LacZ nucleic acid region.

1×10⁶ WiDr cells were injected into the hindlimbs of nude mice and tumors were grown to a mean tumor volume of 260 mm³. Xenografts were injected with 2×10⁸ PFU of Ad5.LacZ or Ad5.Null, two injections per week for a total of four weeks. Control tumors were either not injected or injected with an equivalent volume of buffered saline.

Irradiated mice were immobilized in lucite chambers and the entire body was shielded with lead except for the tumor bearing hindlimb. Tumors were irradiated with 5 Gy per day, 4 days per week, to a total dose of 50 Gy using a Maxitron generator (1.88 Gy/min). Tumor volumes were calculated as before. Data were calculated as the percent of original (day 0) tumor volume and are presented as the fractional tumor volume ±SEM versus days post treatment.

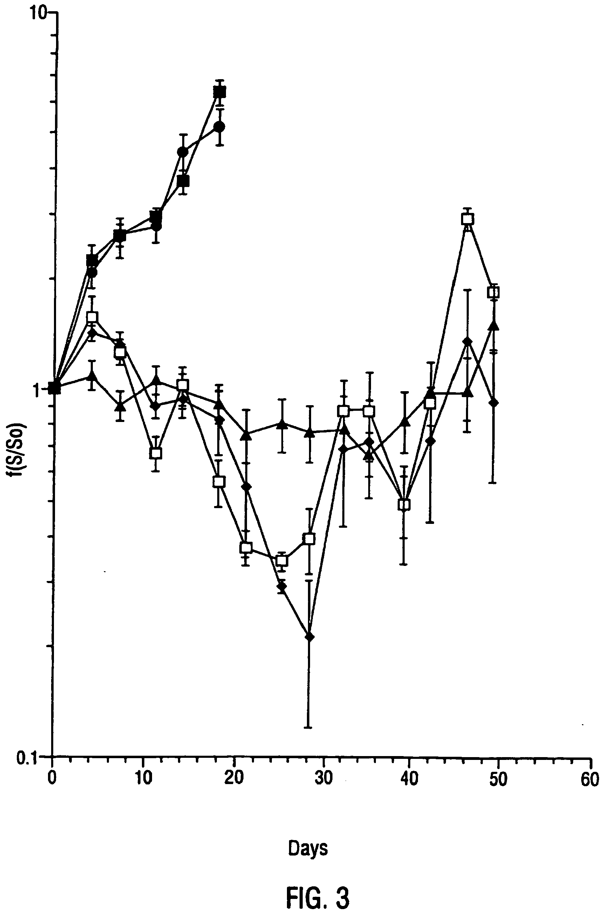

The regression rate of large (>260 mm³) original tumor volume was compared to small (<260 mm³) xenografts following treatment with radiation alone or Ad.LacZ plus radiation. As shown in FIG. 3, tumors treated with Ad.LacZ alone (closed squares) or Ad.null (closed circles) yielded substantially similar tumor growth patterns, requiring the animals to be sacrificed at day 16 when the tumor volume reached greater than 2000 mm³. In contrast, however, tumors treated with Ad.Null, Ad.Egr-TNF or AD.LacZ plus 45 Gy ionizing radiation resulted in tumor volumes remaining relatively constant over a period of 48 days following treatment. Of interest is that by days 22-24, the tumors treated with Ad.nul or Ad.LacZ and radiation showed a significant loss of tumor volume, which eventually regrew to the original volume by days 30-32. The results indicate that it may be possible to re-treat the tumor at between days 22-28 with virus and radiation to further potentiate the synergistic effect of the combination modality.

**Ad CMV Null:** An E1a, partial Elb, partial E3"Ad5 based adenovirus vector that contained an expression cassette replacing the E₁ region consisting of the enhancer/promoter of the immediate-early gene of CMV followed by the SV40 polyadenylation signal and no therapeutic gene. This was used as a control vector in studies of the efficacy of the Ad5.Egr-TNF vector. WiDr cells were injected into the hindlimb of nude mice and grown to a mean tumor volume of 250 mm³. Tumors were then injected with Ad.CMV.Null at 5×108 PFU on days 1, 4, 8, 11 and irradiated with 5 Gy on days 1-4 and 8-11. Tumor volume was measured by calipers twice weekly.

In the studies with the WiDr cell line, the Ad.CMV.Null virus alone had no effect on tumor growth. However, when the Ad.CMV.Null was combined with 45 Gy radiation, tumor regression was started around day 7, and no tumor was seen in 5 out of 12 animals by day 45. In the remaining 7 out of 12 animals, tumor regression reached 6% of original tumor volume by day 28. The tumors had not regrown to their original volume by day 42, indicating that the Ad5 viral vector synergistically enhances *in vivo* radiation tumor control.

### Reference EXAMPLE III

### Treatment Protocols

This prophetic example describes some ways in which the methods of the invention may be used to treat neoplastic disease.
1) Patients exhibiting neoplastic disease are treated a virus for example an adenovirus, at a titer of at between about 10⁸ to about 10¹¹ virus particles, for 6 hours prior to exposure to a DNA damaging agent.
2) Patients are exposed to a DNA damaging agent, e.g. ionizing radiation (2 gy/day for up to 35 days), or an approximate a total dosage of 700 Gy.
3) As an alternative to ionizing radiation exposure, patients are treated with a single intravenous dose of mitomycin C at a dose of 20 mg/m².

It is contemplated that ionizing radiation treatment in combination with a virus, such as an adenovirus, will be effective against cancers of the brain, lung and breast, as well as other neoplasms.

All of the compositions and methods disclosed and claimed herein can be made and executed without undue experimentation in light of the present disclosure. While the compositions and methods of this invention have been described in terms of preferred embodiments, it will be apparent to those of skill in the art that variations may be applied to the composition, methods and in the steps or in the sequence of steps of the method described herein.

### REFERENCES

The following references, to the extent that they provide exemplary procedural or other details supplementary to those set forth herein, are specifically incorporated herein by reference.
Ackermann, M., Chou, J., Sarmiento, M., Lerner, R. A. and Roizman, B., "Identification by antibody to a synthetic peptide of a protein specified by a diploid gene located in the terminal repeats of the L component of herpes simplex virus genome," Journal of Virology, 58:843-50, 1986.
Cassel, W. A. and Garrett, R. E., "Newcastle disease virus as an antineoplastic agent," Cancer (Phila.), 18:863-68, 1965.
Cassel, W. A. and Murray, D. R., "A ten-year follow-up on stage II malignant melanoma patients treated postsurgically with Newcastle disease virus oncolysate," Medical Oncology and Tumor Pharmacotherapy, 9:169-71, 1992.
Chambers, R., "Comparison of genetically engineered HSV," Proc Natl Acad Sci USA, 92:1411-5, 1995.
Chou, J. and Roizman, B., "The gamma 1(34.5) gene of herpes simplex virus 1 precludes neuroblastoma cells from triggering total shutoff of protein synthesis characteristic of programmed cell death in neuronal cells," Proceedings of the National Academy of Sciences of the United States of America, 89:3266-70, 1992.
Chou, J. and Roizman, B., "The herpes simplex virus 1 gene for ICP34.5, which maps in inverted repeats, is conserved in several limited-passage isolates but not in strain 17syn+," Journal of Virology, 64:1014-20, 1990.
Chou, J. and Roizman, B., "The terminal a sequence of the herpes simplex virus genome contains the promoter of a gene located in the repeat sequences of the L component," Journal of Virology, 57:629-37, 1986.
Chou, J., Kern, E. R., Whitley, R. J. and Roizman, B., "Mapping of herpes simplex virus-1 neurovirulence to gamma 134.5, a gene nonessential for growth in culture," Science, 250:1262-6, 1990.
Chou, J., Poon, A. P., Johnson, J. and Roizman, B., "Differential response of human cells to deletions and stop codons in the gamma(1)34.5 gene of herpes simplex virus," Journal of Virology, 68:8304-11, 1994.
Csatary, L. K., "Viruses in the treatment of cancer," Lancet, 2:825, 1971.
Csatary, L. K., Eckhardt, S., Bukosza, I., Czegledi, F., Fenyvesi, C., Gergely, P., Bodey, B. and Csatary, C. M., "Attenuated veterinary vaccine for the treatment of cancer," Cancer Detect. Prev., 17:619-629, 1993.
Culver, K., Ram, Z., Wallbridge, S., Ishii, H., Oldfield, E. and Blaese, R., "In vivo gene transfer with retroviral vector-producer cells for treatment of experimental brain tumors," Science, 256:1550, 1992.
Daumas-Duport, C., Scheithauer, B., O'Fallon, J. and Kelly, P., "Grading of astrocytomas. A simple and reproducible method," Cancer, 62:2152-65, 1988.
Ejercito, P. M., Kieff, E. D. and Roizman, B., "Characterization of herpes simplex virus strains differing in their effects on social behavior of infected cells," Journal of General Virology, 2:357-64, 1968.
Erlich, K. S., Mills, J., Chatis, P., Mertz, G. J., Busch, D. F., Follansbee, S. E., Grant, R. M. and Crumpacker, C. S., "Acyclovir-resistant herpes simplex virus infections in patients with the acquired immunodeficiency syndrome," New England Journal of Medicine 320:293-6, 1989.
Hallahan, D. E., Mauceri, H., Seung, L., Dunphy, E., Wayne, J., Hanna, H., Toledqano, A., Hellman, S., Kufe, D. and Weichselbaum, R. R., "Spatial and temporal control of gene therapy using ionizing radiation," Nature Medicine, 1(8):786-791, 1995.
Jones, N. and Shenk, T., "Isolation of Ad5 mutants defective for transformation," Cell, 17:683-9, 1979.
Kenney, S. and Pagano, J. S., "Viruses as oncolytic agents: a new age for "therapeutic" viruses?" [editorial; comment] Journal of the National Cancer Institute, 86:1185-6, 1994.
Kim, T. S., Halliday, A. L., Hedley-Whyte, E. T. and Convery, K., "Correlates of survival and the Daumas-Duport grading system for astrocytomas," Journal of Neurosurgery, 74:27-37, 1991.
Levin, V. A., Sheline, G. E. and Gutin, P. H., In: Neoplasms of the central nervous system, eds. Devita, V. T., Hellman, S. and Rosenburg, S. (Lippincott, Philadelphia), pp. 1557-1611, 1989.
Lorence, R. M., Katubig, B. B., Reichard, K. W., Reyes, H. M., Phuangsab, A., Sassetti, M. D., Walter, R. J. and Peeples, M. E.,. "Complete regression of human fibrosarcoma xenografts after local Newcastle disease virus therapy," Cancer Research, 54:6017-21, 1994.
Lorence, R. M., Reichard, K. W., Katubig, B. B., Reyes, H. M., Phuangsab, A., Mitchell, B. R., Cascino, C. J., Walter, R. J. and Peeples, M. E., "Complete regression of human neuroblastoma xenografts in athymic mice after local Newcastle disease virus therapy," Journal of the National Cancer Institute, 86:1228-33, 1994.
Mahaley, M., Jr., Mettlin, C., Natarajan, N., Laws, E., Jr. and Peace, B. B., "National survey of patterns of care for brain-tumor patients," Journal of Neurosurgery, 71:826-36, 1989.
Markert, J. M., Malick, A., Coen, D. M. and Martuza, R. L., "Reduction and elimination of encephalitis in an experimental glioma therapy model with attenuated herpes simplex mutants that retain susceptibility to acyclovir," Neurosurgery, 32:597-603, 1993.
Martuza, R. L., Malick, A., Markert, J. M., Ruffner, K. L. and Coen, D. M., "Experimental therapy of human glioma by means of a genetically engineered virus mutant," Science, 252:854-6, 1991.
McGrory, Bautista and Graham, "A simple technique for the rescue of early region I mutations into adenovirus type 5," Virology, 163:614-7, 1988.
Mineta, T., Rabkin, S. D. and Martuza, R. L., "Treatment of malignant gliomas using ganciclovir-hypersensitive, ribonucleotide reductase-deficient herpes simplex viral mutant," Cancer Res, 54:3963-3966, 1994.
Mineta, T., Rabkin, S. D. and Martuza, R. L., "Treatment of malignant gliomas using ganciclovir-hypersensitive, ribonucleotide reductase-deficient herpes simplex viral mutant," Cancer Research, 54:3963-6, 1994.
Ram, Z., Culver, K. W., Walbridge, S., Blaese, R. M. and Oldfield, E. H., "In situ retroviral-mediated gene transfer for the treatment of brain tumors in rats [see comments]," Cancer Research, 53:83-8, 1993(b).
Ram, Z., Culver, K. W., Walbridge, S., Frank, J. A., Blaese, R. M. and Oldfield, E. H., "Toxicity studies of retroviral-mediated gene transfer for the treatment of brain tumors," Journal of Neurosurgery, 79:400-7, 1993(a).
Ram, Z., Walbridge, S., Heiss, J. D., Culver, K. W., Blaese, R. M. and Oldfield, E. H., "In vivo transfer of the human interleukin-2 gene: negative tumoricidal results in experimental brain tumors," Journal of Neurosurgery, 80:535-40, 1994.
Reichard, K. W., Lorence, R. M., Cascino, C. J., Peeples, M. E., Walter, R. J., Fernando, M. B., Reyes, H. M. and Greager, J. A., "Newcastle disease virus selectively kills human tumor cells," Journal of Surgical Research, 52:448-53, 1992.
Reichard, K. W., Lorence, R. M., Katubig, B. B., Peeples, M. E. and Reyes, H. M., "Retinoic acid enhances killing of neuroblastoma cells by Newcastle disease virus," Journal of Pediatric Surgery, 28:1221-5, 1993.
Salazar, O. M., Rubin, P., Feldstein, M. L. and Pizzutiello, R., "High dose radiation therapy in the treatment of malignant gliomas: final report," International Journal of Radiation Oncology, Biology, Physics, 5:1733-40, 1979.
Sibley, G., Hallahan, D., Roizman, B. and Weichselbaum, R., "HSV I vectors for delivery of radiation inducible TNF gene therapy to human glioma xenografts," Int. J. Radiat. Oncol Biol Phys Abstract (In Press), 1995.
Takamiya, Y., Short, M. P., Ezzeddine, Z. D., Moolten, F. L., Breakefield, X. O. and Martuza, R. L., "Gene therapy of malignant brain tumors: a rat glioma line bearing the herpes simplex virus type 1-thymidine kinase gene and wild type retrovirus kills other tumor cells," Journal of Neuroscience Research, 33:493-503, 1992.
Takamiya, Y., Short, M. P., Moolten, F. L., Fleet, C., Mineta, T., Breakefield, X. O. and Martuza, R. L., "An experimental model of retrovirus gene therapy for malignant brain tumors," Journal of Neurosurgery, 79:104-10, 1993.
Walker, M. D., Green, S. B., Byar, D. P., Alexander, E., Jr., Batzdorf, U., Brooks, W. H., Hunt, W. E., MacCarty, C. S., Mahaley, M., Jr., Mealey, J., Jr., Owens, G., Ransohoff, J. d., Robertson, J. T., Shapiro, W. R., Smith, K., Jr., Wilson, C. B. and Strike, T. A., "Randomized comparisons of radiotherapy and nitrosoureas for the treatment of malignant glioma after surgery," New England Journal of Medicine, 303:1323-9, 1980.
Weichselbaum, R., Hallahan, D., Beckett, M., Mauceri, Lee, H., Sukhatme, V. and Kufe, D., "Radiation targeting of gene therapy preferentially radiosensitizes tumor cells," Cancer Research, 54:4266-9, 1994.
Whitley, R. J., Kern, E. R., Chatterjee, S., Chou, J. and Roizman, B., "Replication, establishment of latency, and induced reactivation of herpes simplex virus gamma 1 34.5 deletion mutants in rodent models," Journal of Clinical Investigation, 91, 2837-43, 1993.

## Claims

1. A combination of a herpes simplex virus 1 (HSV-1) and an effective amount of a chemotherapeutic agent which induces DNA damage for simultaneous, separate or sequential use in a method of treatment of the human or animal body.

2. The combination of claim 1, wherein the HSV-1 is non-pathogenic.

3. The combination of claim 2, wherein the HSV-1 is γ₁34.5 minus.

4. The combination of any one of the preceding claims, wherein said herpes simplex virus 1 further contains foreign DNA.

5. The combination of any one of claims 1 to 4, wherein the chemotherapeutic agent is an alkylating agent.

6. The combination of claim 5, wherein said agent is mitomycin C.

7. Use of a herpes simplex virus 1 (HSV-1) for the manufacture of a medicament for the therapy of tumors, wherein the virus is administered simultaneously, separately or sequentially in combination with an effective amount of a chemotherapeutic agent which induces DNA damage.

8. The use of claim 7, wherein the tumor is to be exposed first to the virus and then to be contacted with the chemotherapeutic agent.

9. The use of claim 7, wherein the tumor is to be contacted first with the chemotherapeutic agent and then to be exposed to the virus.

10. The use of any one of claims 7 to 9, wherein the chemotherapeutic agent is an alkylating agent.

11. The use of claim 10, wherein the agent is mitomycin C.

12. The use of any of claims 7 to 11, wherein the tumor is a human or a mouse tumor.

13. The use of claim 12, wherein the tumor is a brain cancer or a breast cancer.

14. The use of any of claims 7 to 13, wherein said virus is to be administered to the tumor by means of an intravenous injection of from about 10⁸ to 10¹¹ virus particles.

15. The use of any of any of claims 7 to 14, wherein said virus is to be administered to the tumor via an oral route.

16. The use of any one of claims 7 to 15, wherein the herpes simplex virus 1 is suitable to be delivered to the tumor site by injection in the form of a pharmaceutical composition.

17. The use of any of claims 7 to 16, wherein the medicament is for potentiating the response of a cell to chemotherapeutic agents.

18. The use of any of claims 7 to 17, wherein the HSV-1 is non-pathogenic

19. The use of claim 18, wherein the HSV-1 is γ₁34.5 minus.

20. The use of any one of claims 7 to 19, wherein the herpes simplex virus 1 further contains foreign DNA.

## Patentansprüche

1. Kombination eines Herpes-Simplex-Virus 1 (HSV-1) und einer wirksamen Menge eines chemotherapeutischen Wirkstoffs, der DNA-Schädigung induziert, zur gleichzeitigen, getrennten oder aufeinanderfolgenden Verwendung in einem Verfahren der Behandlung des menschlichen oder tierischen Körpers.

2. Die Kombination von Anspruch 1, wobei das HSV-1 nicht pathogen ist.

3. Die Kombination von Anspruch 2, wobei das HSV-1 ein γ₁34.5 minus ist.

4. Die Kombination von einem der vorhergehenden Ansprüche, wobei das Herpes-Simplex-Virus 1 weiterhin eine fremde DNA enthält.

5. Die Kombination von einem der Ansprüche 1 bis 4, wobei der chemotherapeutische Wirkstoff ein alkylierender Wirkstoff ist.

6. Die Kombination von Anspruch 5, wobei der Wirkstoff Mitomycin C ist.

7. Verwendung eines Herpes-Simplex-Virus 1 (HSV-1) für die Herstellung eines Medikaments für die Therapie von Tumoren, wobei das Virus gleichzeitig, getrennt oder aufeinanderfolgend in Kombination mit einer wirksamen Menge eines chemotherapeutischen Wirkstoffs, der DNA-Schädigung induziert, verabreicht wird.

8. Die Verwendung von Anspruch 7, wobei der Tumor zuerst gegen das Virus auszusetzen ist und dann mit dem chemotherapeutischen Wirkstoff in Kontakt zu bringen ist.

9. Die Verwendung von Anspruch 7, wobei der Tumor zuerst mit dem chemotherapeutischen Wirkstoff in Kontakt zu bringen ist und dann gegen das Virus auszusetzen ist.

10. Die Verwendung von einem der Ansprüche 7 bis 9, wobei der chemotherapeutische Wirkstoff ein alkylierender Wirkstoff ist.

11. Die Verwendung von Anspruch 10, wobei der Wirkstoff Mitomycin C ist.

12. Die Verwendung von einem der Ansprüche 7 bis 11, wobei der Tumor ein menschlicher Tumor oder ein Maustumor ist.

13. Die Verwendung von Anspruch 12, wobei der Tumor ein Hirnkrebs oder ein Brustkrebs ist.

14. Die Verwendung von einem der Ansprüche 7 bis 13, wobei das Virus an den Tumor mittels einer intravenösen Injektion von etwa 10⁸ bis 10¹¹ Viruspartikeln zu verabreichen ist.

15. Die Verwendung von einem der Ansprüche 7 bis 14, wobei das Virus an den Tumor über einen oralen Weg zu verabreichen ist.

16. Die Verwendung von einem der Ansprüche 7 bis 15, wobei das Herpes-Simplex-Virus geeignet ist, an die Tumorstelle durch Injektion in der Form einer pharmazeutischen Zusammensetzung verabreicht zu werden.

17. Die Verwendung von einem der Ansprüche 7 bis 16, wobei das Medikament zur Potenzierung der Antwort einer Zelle auf chemotherapeutische Wirkstoffe ist.

18. Die Verwendung von einem der Ansprüche 7 bis 17, wobei das HSV-1 nicht pathogen ist.

19. Die Verwendung von Anspruch 18, wobei das HSV-1 γ₁34.5 minus ist.

20. Die Verwendung von einem der Ansprüche 7 bis 19, wobei das Herpes-Simplex-Virus 1 weiterhin fremde DNA enthält.

## Revendications

1. Combinaison d'un virus herpès simplex 1 (HSV-1) et d'une quantité efficace d'un agent chimiothérapeutique qui induit des dommages de l'ADN pour une utilisation simultanée, séparée ou séquentielle dans un procédé de traitement du corps humain ou animal.

2. Combinaison selon la revendication 1, dans laquelle le HSV-1 est non pathogène.

3. Combinaison selon la revendication 2, dans laquelle le HSV-1 est γ₁34.5 moins.

4. Combinaison selon l'une quelconque des revendications précédentes, dans laquelle le virus herpès simplex 1 contient en outre de l'ADN étranger.

5. Combinaison selon l'une quelconque des revendications 1 à 4, dans laquelle l'agent chimiothérapeutique est un agent alkylant.

6. Combinaison selon la revendication 5, dans laquelle ledit agent est la mitomycine C.

7. Utilisation d'un virus herpès simplex 1 (HSV-1) pour la fabrication d'un médicament pour le traitement de tumeurs, dans laquelle le virus est administré simultanément, séparément ou séquentiellement en combinaison avec une quantité efficace d'un agent chimiothérapeutique qui induit des dommages de l'ADN.

8. Utilisation selon la revendication 7, dans laquelle la tumeur doit être exposée en premier au virus et doit ensuite être mise en contact avec l'agent chimiothérapeutique.

9. Utilisation selon la revendication 7, dans laquelle la tumeur doit être mise en contact en premier avec l'agent chimiothérapeutique et doit ensuite être exposée au virus.

10. Utilisation selon l'une quelconque des revendications 7 à 9, dans laquelle l'agent chimiothérapeutique est un agent alkylant.

11. Utilisation selon la revendication 10, dans laquelle l'agent est la mitomycine C.

12. Utilisation selon l'une quelconque des revendications 7 à 11, dans laquelle la tumeur est une tumeur humaine ou une tumeur de souris.

13. Utilisation selon la revendication 12, dans laquelle la tumeur est un cancer du cerveau ou un cancer du sein.

14. Utilisation selon l'une quelconque des revendications 7 à 13, dans laquelle ledit virus doit être administré à la tumeur au moyen d'une injection intraveineuse d'environ 10⁸ à 10¹¹ particules virales.

15. Utilisation selon l'une quelconque des revendications 7 à 14, dans laquelle ledit virus doit être administré à la tumeur par voie orale.

16. Utilisation selon l'une quelconque des revendications 7 à 15, dans laquelle le virus herpès simplex 1 est adapté pour être délivré au site tumoral par injection sous la forme d'une composition pharmaceutique.

17. Utilisation selon l'une quelconque des revendications 7 à 16, dans laquelle le médicament est pour potentialiser la réponse d'une cellule à des agents chimiothérapeutiques.

18. Utilisation selon l'une quelconque des revendications 7 à 17, dans laquelle le HSV-1 est non pathogène.

19. Utilisation selon la revendication 18, dans laquelle le HSV-1 est γ₁34.5 moins.

20. Utilisation selon l'une quelconque des revendications 7 à 19, dans laquelle le virus herpès simplex 1 contient en outre de l'ADN étranger.
